(19) Europäisches Patentamt
European Patent Office
Office européen des brevets

(11) Veröffentlichungsnummer: **0 151 939**
**B1**

# (12) EUROPÄISCHE PATENTSCHRIFT

(45) Veröffentlichungstag der Patentschrift: **30.03.88**

(51) Int. Cl.⁴: **C 07 D 239/54**

(21) Anmeldenummer: **85100296.4**

(22) Anmeldetag: **14.01.85**

(54) **Verfahren zur Herstellung von Pyrimidindionen und Dihydroxypyrimidinen, sowie neue Pyrimidindione und Dihydroxypyrimidine.**

(30) Priorität: **24.01.84 DE 3402193**

(43) Veröffentlichungstag der Anmeldung: **21.08.85 Patentblatt 85/34**

(45) Bekanntmachung des Hinweises auf die Patenterteilung: **30.03.88 Patentblatt 88/13**

(84) Benannte Vertragsstaaten: **BE CH DE FR GB IT LI**

(56) Entgegenhaltungen:

**DE - A - 3 328 154**
**US - A - 4 408 048**

**CHEMICAL ABSTRACTS, Band 54, Nr. 7, 10. April 1960, Columbus, Ohio, USA; E.D. BERGMANN et al. "Organic fluorine compounds. XI. Ethyl fluoroacetates and fluoropyrimidines" und "XII. Preparation and reactions of diethyl fluoromalonate"; Spalten 6736i - 6739g**
**CHEMICAL ABSTRACTS, Band 55, Nr. 24, 27. November 1961, Columbus, Ohio, USA; H. GERSHON et al. "Pyrimidines I. Some halogenated monomethylpyrimidines"; Spalten 24764h - 24765g**
**CHEMICAL ABSTRACTS, Band 55, Nr. 3, 6. Februar 1961, Columbus, Ohio, USA; J.A. CARBON "Synthesis and reactions of 5-bromomethyl- and 5-chloromethyluracil"; Spalten 2658g - 2659c**
**CHEMICAL ABSTRACTS, Band 43, Nr. 6, 25. März 1949, Columbus, Ohio, USA; J.W. CORNFORTH et al. "Action of hydrochloric acid on 5,5-dichloro-6-hydroxy-6-methyl-5,6-dihydrouracil", Spalte 2170b-d**
**THE JOURNAL OF THE AMERICAN CHEMICAL SOCIETY, Band 79, 1957, Easton, Pa., USA; R. DUSCHINSKY et al. "The Synthesis of 5-Fluoropyrimidines", Seiten 4559-4560**

(56) Entgegenhaltungen: (Fortsetzung)

**THE JOURNAL OF THE AMERICAN CHEMICAL SOCIETY, Band 76, 1954, Easton, Pa., USA; R.A. WEST et al. "Synthesis of Chloropyrimidines by Reaction with N-Chlorosuccinimide, and by Condensation Methods", Seiten 3146-3148**
**THE JOURNAL OF THE AMERICAN CHEMICAL SOCIETY, Band 82, 1960 Easton, Pa., USA; J.H. BURCKHALTER et al. "The Amino- und Chloromethylation of Uracil", Seiten 991-994**
**CHEMICAL ABSTRACTS, Band 60, Nr. 11, 25. März 1964, Columbus, Ohio, USA; W. SCHOENAUER "2,5,6-Trichloro-4-methylpyrimidine", Spalte 13257a**
**CHEMICAL ABSTRACTS, Band 74, Nr. 25, 21. Juni 1971, Columbus, Ohio, USA; P. NANTKA-NAMIRSKI et al. "Synthesis of 4-methyluracil derivatives", Seite 582, Zusammenfassung Nr. 141675p**
**CHEMICAL CAPS, Band 67, Nr. 19, 6. November 1967, Columbus, Ohio, USA; R. DOHMORI et al. "Synthesis and antibacterial activity of fluorine-containing pyrimidinylsulfanilamide derivatives", Seite 8545, Zusammenfassung Nr. 90760h**
**BEILSTEINS HANDBUCH DER ORGANISCHEN CHEMIE, 4. Auflage, Band 24; 1936, VERLAG VON JULIUS SPRINGER, Berlin**
**JOURNAL OF FLUORINE CHEMISTRY, Band 21, 1982, Lausanne, CH; E. KLAUKE et al. "Fluorinated heterocyclic compounds: Selective chlorine/fluorine exchange reactions on pyrimidines", Seiten 495-513**
**JOURNAL OF ORGANIC CHEMISTRY, Band 26, November 1961, Washington D.C., USA; S. INOUE et al. "Fluorine-Containing Potential Anticancer Agents. I. Synthesis of Some Trifluoromethylpyrimidines", Seiten 4505-4508**

(73) Patentinhaber: **BAYER AG, Konzernverwaltung RP Patentabteilung, D-5090 Leverkusen 1 Bayerwerk (DE)**

(72) Erfinder: **Baasner, Bernd, Dr., Hamberger Strasse 27d, D-5090 Leverkusen 3 (DE)**
Erfinder: **Klauke, Erich, Dr., Eichendorffweg 8, D-5068 Odenthal (DE)**
Erfinder: **Sasse, Klaus, Dr., Pützweg 13, D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



**EP 0 151 939 B1**

**Beschreibung**

Die vorliegende Erfindung betrifft ein Verfahren zur Herstellung von 2,4-(1H, 3H)-Pyrimidindionen und den dazu tautomeren 2,4-Dihydroxypyrimidinen, sowie von 4,6-Dihydroxypyrimidinen und neue Verbindungen aus diesen Verbindungsklassen.

5-Fluor-2,4-(1H, 3H)-pyrimidindion (=5-Fluoruracil) ist ein bekanntes Cancerostatikum und wird auch als Ausgangsprodukt zur Synthese von Tumorwachstum-inhibierenden Nucleosid-Derivaten verwendet (s. Biomedicinal Aspects of Fluorine Chemistry, Kodansha Ltd., Tokyo, and Elsevier Biomed. Press, Amsterdam–New York–Oxford, 1982, S. 2–5). Die bekannten Synthesen von 5-Fluoruracil über eine Ringschlussreaktion (s. J. Am. Chem. Soc. 79, 4559–4560 (1957)) oder über die Fluorierung von Uracil mit elementarem Fluor (s. US-PS 3,846,429) sind mit erheblichem technischem Aufwand und entsprechend hohen Kosten verbunden. Diese Verfahren sind für eine technische Herstellung nicht geeignet.

In der US-PS 4,299,961 wird eine Herstellung von 5-Fluoruracil beschrieben, bei der man 2,4,5-Trichlorpyrimidin oder 5-Brom-2,4-dichlorpyrimidin mit Metallfluoriden fluoriert, aus dem Fluorierungsgemisch flüchtige Anteile abtrennt und diese hydrolysiert. In dem nach der Hydrolyse vorliegenden Gemisch sind mehrere Uracile enthalten, wobei der Anteil an 5-Fluoruracil zwischen 10 und 40% liegt. Der US-PS 4,299,961 kann nicht entnommen werden, dass 5-Fluoruracil in nahezu 100%iger Selektivität und hoher Ausbeute erhalten werden kann, wenn man reines 2,4,5-Trifluorpyrimidin hydrolysiert.

Weiterhin sind zwei Trifluormethylgruppen enthaltende Pyrimidindione bekannt und von pharmakologischem Interesse, wobei die Synthese dieser Verbindungen bisher durch Fluorierung einer Carbonsäuregruppe mit Schwefeltetrafluorid in Fluorwasserstoff erfolgt (s. J. Pharm. Sci. 52, 508 bis 509 (1963)) oder durch aufwendige Ringschlussreaktionen (s. J. Org. Chem. 26, 4504 bis 4508 (1961)) ausgehend von schwer zugänglichen Ausgangsmaterialien. Beide Herstellungsmethoden sind sehr aufwendig und für eine technische Herstellung ebenfalls nur begrenzt geeignet.

In J.A.C.S., 76, 3146–48 (1954) und 82, 991–94 (1960), sowie in C.A. 60, 13257(a) werden Uracile und Pyrimidine beschrieben, die nicht Gegenstand der vorliegenden Erfindung sind und die im Gegensatz zur vorliegenden Erfindung nicht durch Hydrolyse, sondern durch Chlorierung, teils in Kombination mit einer Hydrierung, hergestellt werden.

Schliesslich wird in der US-PS 4 408 048 die Herstellung von 5-Fluoracil, einem Uracil, das in 6-Position nicht substituiert ist, beschrieben. Da diese Position besonders aktiviert ist und leicht Reaktionen zugänglich ist, war nicht zu erwarten, dass es gelingen könnte, Substituenten in 2- und 4-Position durch Hydrolyse zu entfernen und dabei Substituenten in 6-Postition nicht abzuspalten.

Es wurde nun ein Verfahren zur Herstellung von Pyrimidindionen und Dihydroxypyrimidinen der Formeln

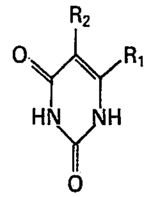
(I)

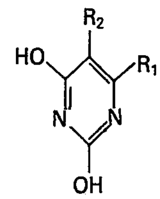
(II)

und

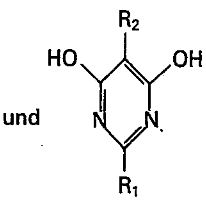
(III)

in denen

$R_1$ für Fluor, Chlor oder eine $C_1$- bis $C_4$-Alkylgruppe steht, wobei die Wasserstoffatome der Alkylgruppe vollständig oder teilweise durch Fluor und/oder Chlor substituiert sind und $R_2$ für Wasserstoff, Fluor oder Chlor und im Falle $R_1$=Fluor oder Chlor auch für eine $C_1$- bis $C_4$-Alkylgruppe steht, wobei die Wasserstoffatome der Alkylgruppe gegebenenfalls vollständig oder teilweise durch Fluor und/oder Chlor substituiert sein können, gefunden, das dadurch gekennzeichnet ist, dass man Fluor und/oder Chlor enthaltende Pyrimidine der Formeln

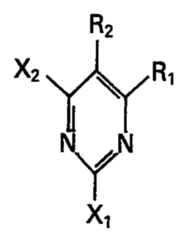
(IV)

oder

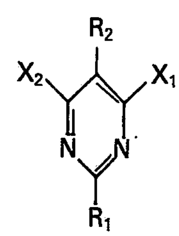
(V)

in denen

$R_1$ und $R_2$ die oben angegebene Bedeutung haben und

$X_1$ und $X_2$ unabhängig voneinander für Fluor oder Chlor stehen, mit wässrigen Säuren oder Alkalien hydrolysiert.

Die Ausgangsprodukte für das erfindungsgemässe Verfahren sind gut zugänglich. Verbindungen der Formel (IV) können besonders gut durch selektive katalytische Hydrierung von Chlor enthaltenden Pyrimidinen erhalten werden, wobei die Hydrierung in Gegenwart von Chlorwasserstoff-Akzeptoren durchgeführt wird. Verbindungen der Formel (V) können besonders gut durch Umsetzung von mit Fluor, Chlor und/oder $CF_3$ substituierten Pyrimidinen mit Chlorwasserstoff erhalten werden (s. DE-OS 33 28 154). Einige Ausgangsverbindungen können auch gemäss J. Fluorine Chem. 21, 495–513 (1982) erhalten werden.

Zum Einsatz in das erfindungsgemässe Verfahren sind Pyrimidine der Formeln (IV) und (V) bevorzugt, bei denen $R_1$ für Fluor, Chlor, $CF_3$, $CF_2Cl$, $CCl_2F$, $CCl_3$, $CHCl_2$, $CHF_2$, $CHClF$, $CH_2F$ oder $CH_2Cl$ und $R_2$ für Wasserstoff, Fluor oder Chlor und im Falle $R_1$=Fluor oder Chlor auch für $CF_3$, $CF_2Cl$,

$CCl_2F$, $CCl_3$, $CHCl_2$, CHClF, $CH_2F$ oder $CH_2Cl$ steht und $X_1$ und $X_2$ unabhängig voneinander für Fluor oder Chlor stehen.

Besonders bevorzugt zum Einsatz in das erfindungsgemässe Verfahren sind Pyrimidine der Formeln (IV) und (V), bei denen $R_1$ für Fluor, Chlor oder $CF_3$ und $R_2$ für Wasserstoff, Fluor oder Chlor und im Falle von $R_1$=Fluor oder Chlor auch für $CF_3$ steht und $X_1$ und $X_2$ unabhängig voneinander für Fluor oder Chlor stehen.

Ganz besonders werden in das erfindungsgemässe Verfahren 2,4,5-Trifluor-, 2,4-Dichlor-5-fluor-, 2,5-Difluor-4-chlor-, 2,4,6-Trichlor-5-fluor-, 2,5-Difluor-4,6-Dichlor-, 2,4,5-Trifluor-6-chlor-, 2,4,6-Trifluor-, 2,4,5-Trifluor-6-trifluormethyl-, 2,5-Difluor-4-chlor-6-trifluormethyl-, 2,4-Dichlor-5-fluor-6-trifluormethyl-, 2,4,5-Trichlor-6-trifluormethyl-, 2,5-Dichlor-4-fluor-6-trifluormethyl-, 2,4,6-Trifluor-5-trifluormethyl-, 2,4,6-Trifluor-5-chlormethyl-, 2,4,6-Trifluor-5-dichlormethyl-, 2,4,6-Trifluor-5-trichlormethyl-, 2,4,6-Trifluor-5-difluormethyl-, 2,4,6-Trifluor-5-difluorchlormethyl-, 2,4,6-Trifluor-5-fluordichlormetyhl-, 2,4,6-Trifluor-5-fluorchlormethyl-, 2,4,5-Trichlormethyl-6-dichlormethyl-, 2,4-Difluor-5-chlor-6-dichlormethyl-, 2,5-Dichlor-4-fluor-6-dichlormethyl-, 2-Fluor-4,5-difluor-6-dichlormethyl-, 2-Fluor-4,5-dichlor-6-methyl-, 2,4,5-Trichlor-6-fluordichlormethyl-, 2,4,5-Trichlor-6-difluorchlormethyl-, 2,4-Difluor-5-chlor-6-difluorchlormethyl-,2,4-Difluor-5-chlor-6-fluordichlormethyl, 2,4-Difluor-5-chlor-6-fluorchlormethyl-, 2-Trifluormethyl-4,5,6-trifluormethyl-, 2-Trifluormethyl-4,5-difluor-6-chlor-, 2-Trifluormethyl-4,6-dichlor-5-fluor-, 2-Trichlormethyl-4,6-difluor-5-chlor-, 2,4-Difluor-5-trifluormethyl-, 2,4-Difluor-5-trifluormethyl- und 2-Fluor-4-chlor-5-trifluormethylpyrimidin eingesetzt.

Die erfindungsgemässe Hydrolyse kann beispielsweise im Temperaturbereich von 0°C bis zum Siedepunkt des Reaktionsgemisches durchgeführt werden, der je nach der verwendeten Säure, dem verwendeten Alkali oder dem gegebenenfalls eingesetzten Lösungsmittel bis zu etwa 160°C betragen kann. Bevorzugt sind Temperaturen im Bereich von 40 bis 140°C, besonders bevorzugt solche im Bereich von 50 bis 100°C. Wird die erfindungsgemässe Hydrolyse im alkalischen Bereich durchgeführt, so arbeitet man vorzugsweise bei Temperaturen unter 100°C.

Die erfindungsgemässe Hydrolyse wird im allgemeinen bei Normaldruck durchgeführt. Sie kann jedoch auch bei erniedrigtem oder erhöhtem Druck durchgeführt werden, beispielsweise in einem Rührautoklaven und gegebenenfalls unter Druckerhöhung durch ein aufgedrücktes Inertgas, z. B. Stickstoff.

In die erfindungsgemässe Hydrolyse werden neben Ausgangsprodukt und Wasser entweder Säuren oder Alkalien eingesetzt. Prinzipiell können hierfür beliebige Säuren und Alkalien verwendet werden. Vorzugsweise wird als Säure Schwefelsäure, Salzsäure, Essigsäure, Salpetersäure oder Phosphorsäure verwendet, besonders bevorzugt Schwefelsäure.

Als Alkalien werden vorzugsweise Natronlauge oder Kalilauge verwendet.

Die Mengen an Wasser und Säuren oder Alkalien werden im allgemeinen so gewählt, dass mindestens 2 Mole Wasser und mindestens 2 Mole Säure oder Alkali pro Mol Ausgangsmaterial eingesetzt werden. Vorzugsweise werden pro Mol Ausgangsmaterial 2 bis 100 Mole Wasser und 2 bis 4 Mole Säure oder Alkali eingesetzt. Höhere Überschüsse an Wasser und Säure oder Alkali sind möglich, jedoch im allgemeinen ohne besonderen Effekt. Die eingesetzten Säuren oder Alkalien können von beliebiger Konzentration sein, beispielsweise können Säuren in Form von 5 bis 95 Gew.-%igen und Alkalien in Form von 3 bis 50 Gew.-%igen wässrigen Lösungen eingesetzt werden.

Die Zugabe von Wasser und Säuren oder Alkalien kann auf verschiedene Weise erfolgen. Beispielsweise kann man das Ausgangsmaterial in Wasser suspendieren und die Säure oder das Alkali in Form konzentrierter Lösungen langsam zugeben. Man kann auch das Ausgangsmaterial direkt mit wässriger Säure oder Alkali versetzen und gegebenenfalls danach die gewünschte Reaktionstemperatur einstellen.

Zur Erleichterung des Angriffs der wässrigen Säure oder des wässrigen Alkali auf das Ausgangsmaterial kann es vorteilhaft sein, die Hydrolyse in Gegenwart eines inerten organischen Lösungsmittels durchzuführen. Geeignete Lösungsmittel sind beispielsweise Dioxan, Tetrahydrofuran, Aceton, Dimethylformamid und Diethylenglykoldimethylether. Die Reaktion kann dann beispielsweise so durchgeführt werden, dass man das Ausgangsmaterial in dem Lösungsmittel löst oder suspendiert und zu dieser Lösung oder Suspension eine wässrige Lösung von Säuren oder Alkalien hinzufügt. In Gegenwart von entsprechenden Lösungsmitteln kann das erfindungsgemässe Verfahren auch bei Temperaturen unter 0°C oder ohne Druckerhöhung oberhalb des Siedepunktes des lösungsmittelfreien Reaktionsgemisches durchgeführt werden. Im allgemeinen werden hierdurch jedoch keine besonderen Vorteile erzielt, denn unter 0°C ist die Reaktionsgeschwindigkeit im allgemeinen recht gering, und bei hohen Temperaturen kann die Gefahr von Zersetzungen der Ausgangs- und/oder Reaktionsprodukte bestehen.

Die erfindungsgemässe Hydrolyse benötigt im allgemeinen eine Reaktionszeit im Bereich von 0,5 bis 24 Stunden. Häufig kann sie in 0,5 bis 6 Stunden zu Ende geführt werden.

Das nach der Hydrolyse vorliegende Gemisch kann auf verschiedene Weise aufgearbeitet werden. Beispielsweise kann man das Reaktionsprodukt mit einem inerten, polaren Lösungsmittel wie Diethylether oder Dichlormethan aus dem Reaktionsgemisch extrahieren und nach Abdestillieren des Extraktionsmittels gewinnen. Man kann auch zunächst das Reaktionsgmisch neutralisieren, dann flüchtige Bestandteile abfiltrieren und aus dem festen Rückstand die anorganischen Salze mit Wasser auswaschen, wobei das Reaktionsprodukt zurückbleibt. In einigen Fällen kann man das Reaktionsprodukt auch dadurch gewinnen, dass man es bei tiefen Temperaturen, z. B. bei 0 bis 5°C, aus dem Reaktionsgemisch auskristallisieren oder durch Eingiessen in Eiswasser sich abscheiden lässt.

Das auf eine der vorstehend beschriebenen Arbeitsweisen oder auf sonstige Weise gewonnene Reaktionsprodukt kann gegebenenfalls weiter gereinigt werden, beispielsweise durch Umkristallisation oder Sublimation.

Aus Ausgangsmaterialien der Formel (IV) wird im allgemeinen ein Gemisch der entsprechenden Pyrimidindione und Dihydroxypyrimidine der Formel (I) und (II) erhalten. Diese beiden Stoffe liegen in einem tautomeren Gleichgewicht vor, das bei Raumtemperatur ganz überwiegend auf der Seite der Pyrimidindione der Formel (I) liegt. Durch Temperaturerhöhung kann dieses Gleichgewicht in Richtung auf die Dihydroxypyrimidine der Formel (II) verschoben werden. Aus Ausgangsmaterialien der Formel (V) werden die entsprechenden Dihydroxypyrimidine der Formel (III) erhalten. Eine Tautomerie tritt hier nicht auf.

Die Pyrimidindione und Dihydroxypyrimidine der Formeln (I), (II) und (III) sind zum grössten Teil neue Stoffe. Sowohl die bekannten Stoffe, das sind die Pyrimidindione der Formel (I) mit $R_1$ und $R_2$=Fluor, mit $R_1=CF_3$ und $R_2$=Wasserstoff und mit $R_1$=Wasserstoff und $R_2=CF_3$, als auch die neuen Stoffe sind Cancerostatika, Vorprodukte für die Synthese von Tumorwachstum-inhibierenden Wirkstoffen, Pflanzenschutzwirkstoffe und Vorprodukte für Pflanzenschutzwirkstoffe.

Im allgemeinen werden bei der erfindungsgemässen alkalischen und sauren Hydrolyse vergleichbare Ergebnisse erhalten. Lediglich, wenn das Ausgangsmaterial $CCl_3$-Gruppen enthält, ist die alkalische Hydrolyse wenig geeignet, um zu guten Ergebnissen zu kommen. Hier ist die saure Hydrolyse vorzuziehen.

Mit dem erfindungsgemässen Verfahren gelingt es überraschenderweise, bisher nur schwer oder überhaupt nicht zugängliche Pyrimidindione und Dihydroxypyrimidine auf einfache Weise und in hohen Ausbeuten und Selektivitäten zu erhalten. Häufig verläuft die erfindungsgemässe Hydrolyse nahezu quantitativ, d.h. Produkteverluste treten häufig nur durch die Aufarbeitung und gegebenenfalls durch die Reinigung auf.

Die vorliegende Erfindung betrifft weiterhin neue Pyrimidindione und Dihydroxypyrimidine der Formeln

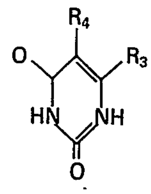
(VI)

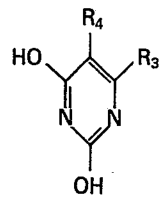
(VII)

und

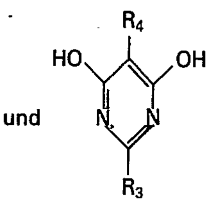
(VIII)

in denen einer der Reste $R_3$ und $R_4$ für Fluor, Chlor, $CF_3$, $CF_2Cl$, $CCl_2F$, $CHCl_2$, $CHF_2$ oder $CHClF$ und $R_4$ für Fluor oder Chlor steht, wobei die Verbindungen ausgenommen sind, bei denen $R_3$ für $CF_3$ und $R_4$ für Fluor steht.

Herstellungs- und Anwendungsmöglichkeiten für die neuen Verbindungen der Formeln (VI), (VII)

Tabelle 1

| Beisp. Nr. | Ausgangsprodukt 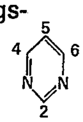 | erhalten gemäss | Reaktionsprodukt 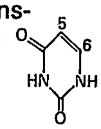 | Ausbeute (% der Theorie) | Schmelzpunkt (°C) |
|---|---|---|---|---|---|
| 2 | 6-Trifluormethyl-5-fluor-2,4-dichlor- | DE-OS 33 28 154 Beispiel 3 | 6-Trifluormethyl-5-fluor- | 78 | 224–226 |
| 3 | 6-Trifluormethyl-2,4,5-trifluor- | J. Fluorine Chem. 21, 495 (1982) | 6-Trifluormethyl-5-fluor- | 93 | 224–225 |
| 4 | 5-Trifluormethyl-2,4,6-trifluor- | J. Chem. Soc. C, 1967, 1822. | 5-Trifluormethyl-6-fluor- | 57 | über 250 |
| 5 | 2,4-Difluor-5-chlor-6-dichlor-methyl- | J. Fluorine Chem. 21, 495 (1982) | 5-Chlor-6-dichlor-methyl- | 54 | über 260 |
| 6 | 2,4-Difluor-5-chlor-6-methyl- | J. Fluorine Chem. 21, 495 (1982) | 5-Chlor-6-methyl- | 74 | über 250 |
| 7 | 2-Fluor-4,5-dichlor-6-methyl- | J. Fluorine Chem. 21, 495 (1982) | 5-Chlor-6-methyl- | 93 | über 250 |
| 8 | 2,4,5-Trichlor-6-methyl- | J. Fluorine Chem. 21, 495 (1982) | 5-Chlor-6-methyl- | 89 | über 250 |
| 9 | 2,4,6-Trifluor-5-difluormethyl- | J. Fluorine Chem. 21, 495 (1982) | 5-Difluormethyl-6-fluor- | 72 | über 250 |
| 10 | 2,4,6-Trifluor-5-chlor methyl- | J. Fluorine Chem. 21, 495 (1982) | 5-Chlormethyl-6-fluor | 83 | über 250 |

und (VIII) sind bereits weiter oben beschrieben worden.

Beispiel 1

218,5g (1 Mol) 2,5-Difluor-4-chlor-6-trifluormethylpyrimidin (erhalten gemäss Beispiel 3 der DE-OS 3328154) wurden in 1,5l Wasser aufgenommen und unter Rühren auf 80°C (Innentemperatur) erhitzt. Bereits während des Aufheizens wurde damit begonnen, 200g 45Gew.-%ige wässrige Natronlauge zuzutropfen. Nach beendeter Zugabe wurde 4 Stunden bei 80°C nachgerührt. Nach dem Erkalten wurde mit konzentrierter Salzsäure neutralisiert und der ausgefallene Feststoff abgesaugt. Dieser Feststoff wurde mit 500 ml Wasser digeriert, erneut abgesaugt und bis zur Wasserfreiheit bei 50°C im Vakuum getrocknet. Es wurden 176g (89% der Theorie) 6-Trifluormethyl-5-fluorpyrimidindion mit einem Schmelzpunkt von 224 bis 225°C erhalten.

Beispiele 2 bis 10

Es wurde verfahren wie in Beispiel 1, jedoch wurden andere Ausgangsprodukte eingesetzt. Die eingesetzten Ausgangsprodukte, die erhaltenen Reaktionsprodukte und sonstige Kenndaten sind in Tabelle 1 zusammengefasst.

Beispiel 11

17g 2,4,5-Trichlor-6-trifluormethylpyrimidin (erhalten gemäss J. Fluorine Chem. 21, 495 bis 513 (1982) wurden mit 100ml wässriger 70Gew.-%-iger Schwefelsäure versetzt und 5 Stunden lang auf 140°C erhitzt. Danach wurde das Raktionsgemisch in Eiswasser gegossen und abgesaugt. Nach Umkristallisation des abfiltrierten Festprodukts aus Essigsäureethylester wurden 8g 5-Chlor-6-trifluor-2,4-(1H,3H)-pyrimidindion mit einem Schmelzpunkt von 228 bis 230°C erhalten.

Beispiele 12 bis 18

Es wurde verfahren wie in Beispiel 11, jedoch wurden andere Ausgangsprodukte eingesetzt. Die eingesetzten Ausgangsprodukte, die erhaltenen Reaktionsprodukte und sonstige Kenndaten sind in Tabelle 2 zusammengefasst.

Beispiel 19

Es wurde verfahren wie in Beispiel 1, jedoch wurde 2-Trifluormethyl-4,5,6-trifluorpyrimidin eingesetzt und 2-Trifluormethyl-5-fluor-4,6-dihydroxypyrimidin eingesetzt in 83%iger Ausbeute mit einem Schmelzpunkt von über 250°C erhalten.

Beispiel 20

Es wurde verfahren wie in Beispiel 1, jedoch wurde 2-Trifluormethyl-5-fluor-4,6-dichlorpyrimidin eingesetzt und 2-Trifluormethyl-5-fluor-4,6-dihydroxypyrimidin in 76%iger Ausbeute mit einem Schmelzpunkt von über 250°C erhalten.

Beispiel 21

Es wurde verfahren wie in Beispiel 1, jedoch wurde 2-Trifluormethyl-4-chlor-5,6-difluorpyrimidin eingesetzt und 2-Trifluormethyl-5-fluor-4,6-dihydroxypyrimidin in 87%iger Ausbeute mit einem Schmelzpunkt von über 250°C erhalten.

Beispiel 22

Es wurde verfahren wie in Beispiel 11, jedoch wurde 2-Trichlormethyl-4,6-difluor-5-chlorpyrimidin eingesetzt und 2-Trichlormethyl-5-chlor-4,6-di-

Tabelle 2

| Beisp. Nr. | Ausgangsprodukt 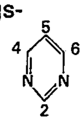 | erhalten gemäss | Reaktionsprodukt 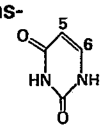 | Ausbeute (% der Theorie) | Schmelzpunkt (°C) |
|---|---|---|---|---|---|
| 12 | 6-Trifluormethyl-2,4-5-trifluor- | J. Fluorine Chem. 21, 495–513 (1982) | 6-Trifluormethyl-5-fluor- | 61 | 222–224 |
| 13 | 2,4-Difluor-5-chlor-6-difluor-chlormethyl- | J. Fluorine Chem. 21, 495–513 (1982) | 5-Chlor-6-difluor-chlormethyl- | 68 | 218–220 |
| 14 | 2,4-Difluor-5-chlor-6-dichlorfluormethyl- | J. Fluorine Chem. 21, 495–513 (1982) | 5-Chlor-6-dichlor-fluormethyl- | 64 | 215–217 |
| 15 | 2,4,5-Trichlor-6-dichlorfluormethyl- | J. Fluorine Chem. 21, 495–513 (1982) | 5-Chlor-6-dichlor-fluormethyl- | 73 | 217 |
| 16 | 2,4,5-Trichlor-6-difluorchlormethyl- | J. Fluorine Chem. 21, 495–513 (1982) | 5-Chlor-6-difluor-chlormethyl- | 77 | 217–219 |
| 17 | 2,4-Dichlor-5-fluor-6-trifluormethyl- | DE-OS 2328154 | 5-Fluor-6-trifluor-methyl- | 71 | 220–224 |
| 18 | 2,4-Difluor-5-chlor-6-dichlormethyl- | J. Fluorine Chem. 21, 495–513 (1982) | 5-Chlor-6-dichlor-methyl | 81 | über 250 |

hydroxypyrimidin in 68%iger Ausbeute mit einem Schmelzpunkt von über 250°C erhalten.

Die Ausgangsprodukte der Beispiele 19 bis 22 wurden nach DE-OS 23 28 154 erhalten.

Beispiel 23

(Charakterisierung der Reaktionsprodukte der Beispiele 1 bis 22)

a) Die Charakterisierung aller Substanzen erfolgte $^{19}F$-, $^{13}C$-, $^{1}H$-NMR- und IR-spektroskopisch, sowie durch Massenspektrometrie.

Alle 2,4-(1H,3H)-pyrimidindione zeigen bei IR-Aufnahmen die für derartige Verbindungen typischen Carbonylfrequenzen. Dabei wurden die Messungen sowohl im KBr-Pressling als auch in einem Lösungsmittel (DMSO-$d_6$) durchgeführt.

b) Durch $^{13}C$-NMR-Messungen wurde nachgewiesen, dass das 5-Fluor-6-trifluormethyl-2,4-(1H,3H)-pyrimidindion, unter den Messbedingungen nach dem IR-Spektrum und dem $^{13}C$-Spektrum ausschliesslich in der Dion-Form

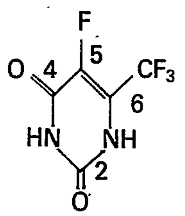

und nicht in der Dihydroxy-Form vorliegt.

Tabelle 3

$^{13}C$-Daten von 5-Fluor-6-trifluormethyl-2,4-(1H,3H)-pyrimidindion in DMSO-$d_6$, gemessen bei 50,3MHz und 16°C.

| C-Atome | δ[ppm] | $^{13}C$-$^{19}F$-Kopplungen [Hz] |
|---|---|---|
| C-2 | 149.727 | keine |
| C-4 | 157.461 | $^2J$= 25.8 |
| C-5 | 138.222 | $^1J$=248.2 |
| C-6 | 126.576 | $^2J$= 17.1 und 35.7 |
| $CF_3$ an C-6 | 119.573 | $^1J$=275.6; $^3J$=3.3 |

c) Das 5-Chlor-6-methyl-2,4-(1H,3H)-pyrimidindion (A) steht dagegen unter den Messbedingungen (s. Tabelle 4) mit seiner tautomeren Dihydroxy-Form, dem 5-Chlor-6-methyl-2,4-dihydroxypyrimidin (B) im Gleichgewicht gemäss:

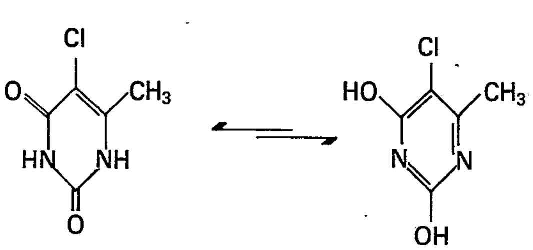

(A) (B)

Das Verhältnis von (A) zu (B) betrug unter den Messbedingungen (s. Tabelle 4) 95:5 und war temperaturabhängig. Die übrigen hergestellten neuen Pyrimidindione verhielten sich analog.

Tabelle 4

$^{13}C$-Daten von (A) und (B) in DMSO-$d_6$, gemessen bei 50,3 MHz und 16°C.

| C-Atome | (A) δ [ppm] | (B) δ [ppm] |
|---|---|---|
| C-2 | 149.879 | 164.557 |
| C-4 | 159.636 | 153.359 |
| C-5 | 104.514 | 99.158 |
| C-6 | 149.144 | 152.034 |
| $CH_3$ an C-6 | 16.801 | 18.188 |

Das Verhältnis von (A) zu (B) wurde durch Integration der jeweiligen Signalgruppen bestimmt.

b) Die Verbindungen aus den Beispielen 19 bis 22 liegen nur in der Dihydroxy-Form vor. Das geht eindeutig aus den $^{13}C$-NMR-Daten von 2-Trifluormethyl-5-fluor-4,6-dihydroxyprymidin hervor:

Tabelle 5

$^{13}C$-Daten von 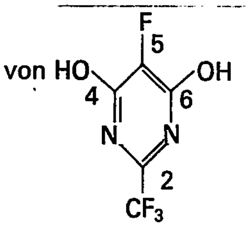

in DMSO-$d_6$ bei 50,3 MHz und 16°C.

| C-Atome | δ [ppm] | $^{13}C$-$^{19}F$-Kopplungen [Hz] |
|---|---|---|
| C-2 | 146.831 | $^2J$= 36.6; $^4J$=10.3 |
| $CF_3$ an C-2 | 118.896 | $^1J$=275.5 |
| C-4/C-6 | 159.042 | $^2J$= 10.0 |
| C-5 | 131.817 | $^1J$=255.2 |

Beispiel 24

Pre-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert.

Die Ergebnisse sind aus Tabelle 6 ersichtlich.

Tabelle 6

| Wirkstoff | Wirkstoffaufwand | Wirkung |
|---|---|---|
| Produkt aus Beispiel 3 und 12 | 2 kg/ha | Senf und Hafer werden zum grösseren Teil vernichtet; Weizen und Mais fast nicht. |
| Produkt aus Beispiel 13 | 2 kg/ha | Senf und Rüben werden nahezu vollständig vernichtet; Weizen, Hafer und Mais praktisch nicht. |
| Produkt aus Beispiel 14 | 2 kg/ha | Senf wird zum grösseren Teil vernichtet; Weizen, Hafer, Hirse und Mais praktisch nicht. |

Beispiel 25
Post-emergence-Test
Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben, so dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert.

Die Ergebnisse sind aus Tabelle 7 ersichtlich.

Tabelle 7

| Wirkstoff | Wirkstoffaufwand | Wirkung |
|---|---|---|
| Produkt aus Beispiel 11 | 1 kg/ha | Senf, Bohnen, Baumwolle und Mais werden vollständig oder nahezu vollständig vernichtet; Weizen, Hafer und Rüben praktisch nicht. |
| Produkt aus Beispiel 3 und 12 | 1 kg/ha | Senf und Rüben werden vollständig oder nahezu vollständig vernichtet; Weizen praktisch nicht. |
| Produkt aus Beispiel 13 | 1 kg/ha | Senf wird vollständig vernichtet; Mais, Weizen und Hafer praktisch nicht. |

**Patentansprüche**

1. Verfahren zur Herstellung von Pyrimidindionen und Dihydroxypyrimidinen der Formeln

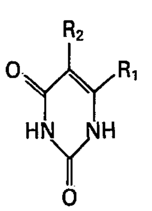
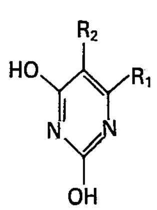
und
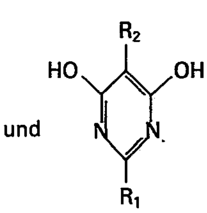

in denen
$R_1$ für Fluor, Chlor oder eine $C_1$- bis $C_4$-Alkylgruppe steht, wobei die Wasserstoffatome der Alkylgruppe vollständig oder teilweise durch Fluor und/oder Chlor substituiert sind und
$R_2$ für Wasserstoff, Fluor oder Chlor und im Falle $R_1$=Fluor oder Chlor auch für eine $C_1$- bis $C_4$-Alkylgruppe steht, wobei die Wasserstoffatome der Alkylgruppe gegebenenfalls vollständig oder teilweise durch Fluor und/oder Chlor substituiert sein können, dadurch gekennzeichnet, dass man Fluor und/oder Chlor enthaltende Pyrimidine der Formeln

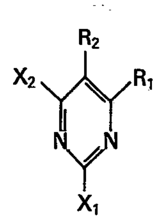
oder
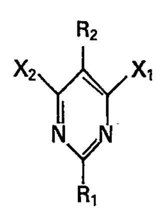

in denen
$R_1$ und $R_2$ die oben angegebene Bedeutung haben und $X_1$ und $X_2$ unabhängig voneinander für

Fluor oder Chlor stehen, mit wässrigen Säuren oder Alkalien hydrolysiert.

2. Verfahren nach Anspruch 1, dadurch gekennzeichnet, dass man Pyrimidine der angegebenen Formeln einsetzt, bei denen $R_1$ für Fluor, Chlor, $CF_3$, $CF_2Cl$, $CCl_2F$, $CCl_3$, $CHCl_2$, $CHF_2$, $CHClF$, $CH_2F$ oder $CH_2Cl$ und $R_2$ für Wasserstoff, Fluor oder Chlor und im Falle von $R_1$=Fluor oder Chlor auch für $CF_3$, $CF_2Cl$, $CCl_2$-F, $CCl_3$, $CH_3$, $CHCl_2$, $CHF_2$, $CHClF$, $CH_2F$ oder $CH_2Cl$ steht und $X_1$ und $X_2$ unabhängig voneinander für Chlor stehen.

3. Verfahren nach Ansprüchen 1 und 2, dadurch gekennzeichnet, dass man Pyrimidine der angegebenen Formeln einsetzt, bei denen $R_1$ für Fluor, Chlor oder $CF_3$ und $R_2$ für Wasserstoff, Fluor oder Chlor und im Falle von $R_1$=Fluor oder Chlor auch für $CF_3$ steht und $X_1$ und $X_2$ unabhängig voneinander für Fluor oder Chlor stehen.

4. Verfahren nach Ansprüchen 1 bis 3, dadurch gekennzeichnet, dass man es bei Temperaturen von 0°C bis 160°C durchführt.

5. Verfahren nach Ansprüchen 1 bis 4, dadurch gekennzeichnet, dass man es unter Zugabe von Schwefelsäure, Salzsäure, Essigsäure, Salpetersäure, Phosphorsäure, Natronlauge oder Kalilauge durchführt.

6. Verfahren nach Ansprüchen 1 bis 5, dadurch gekennzeichnet, dass man mindestens 2 Mole Wasser und mindestens 2 Mole Säure oder Alkali pro Mol Ausgangsmaterial einsetzt.

7. Verfahren nach Ansprüchen 1 bis 6, dadurch gekennzeichnet, dass man es in Gegenwart eines inerten organischen Lösungsmittels durchführt.

8. Pyrimidindione und Dihydroxypyrimidine der Formeln

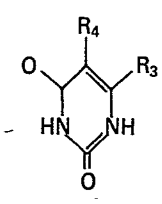

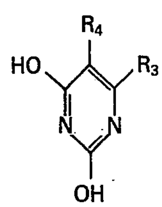

und

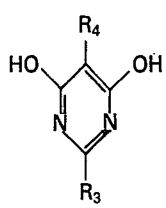

in denen

einer der Reste $R_3$ und $R_4$ für Fluor, Chlor, $CF_3$, $CF_2Cl$, $CCl_2F$, $CHCl_2$, $CHF_2$ oder $CHClF$ und der andere der Reste $R_3$ und $R_4$ für Fluor oder Chlor steht, wobei die Verbindungen ausgenommen sind, bei denen $R_3$ für $CF_3$ und $R_4$ für Fluor steht.

**Claims**

1. Process for the preparation of pyrimidinediones and dihydroxypyrimidines of the formulae

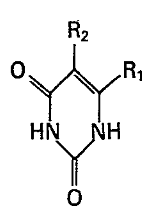

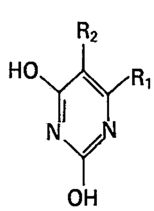

and

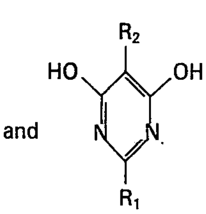

in which

$R_1$ represents fluorine, chlorine or a $C_1$- to $C_4$-alkyl group, and some or all of the hydrogen atoms of the alkyl group are substituted by fluorine and/or chlorine, and $R_2$ represents hydrogen, fluorine or chlorine, and, in the case where $R_1$=fluorine or chlorine, also represents a $C_1$- to $C_4$-alkyl group, and some or all of the hydrogen atoms of the alkyl group can optionally be substituted by fluorine and/or chlorine, characterised in that pyrimidines containing fluorine and/or chlorine, of the formulea

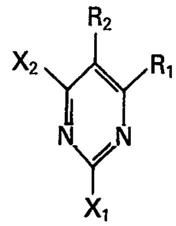

or

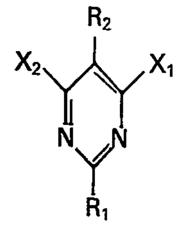

in which

$R_1$ and $R_2$ have the above meaning and $X_1$ and $X_2$ independently of one another represent fluorine or chlorine, are hydrolysed with aqueous acids or alkalis.

2. Process according to Claim 1, characterised in that pyrimidines of the formulae given are employed, in which $R_1$ represents fluorine, chlorine, $CF_3$, $CF_2Cl$, $CCl_2F$, $CCl_3$, $CHCl_2$, $CHF_2$, $CHClF$, $CH_2F$ or $CH_2Cl$, and $R_2$ represents hydrogen, fluorine or chlorine, and, in the case where $R_1$=fluorine or chlorine, also represents $CF_3$, $CF_2Cl$, $CCl_2$-F, $CCl_3$, $CH_3$, $CHCl_2$, $CHF_2$, $CHClF$, $CH_2F$ or $CH_2Cl$, and $X_1$ and $X_2$ independently of one another represent chlorine.

3. Process according to Claims 1 and 2, characterised in that pyrimidines of the formulae given are employed, in which $R_1$ represents fluorine, chlorine or $CF_3$, and $R_2$ represents hydrogen, fluorine or chlorine, and, in the case where $R_1$= fluorine or chlorine, also represents $CF_3$, and $X_1$ and $X_2$ independently of one another represent fluorine or chlorine.

4. Process according to Claims 1 to 3, characterised in that it is carried out at temperatures from 0°C to 160°C.

5. Process according to Claims 1 to 4, characterised in that it is carried out with the addition of sulphuric acid, hydrochloric acid, acetic acid, nitric acid, phosphoric acid, sodium hydroxide solution or potassium hydroxide solution.

6. Process according to Claims 1 to 5, characterised in that at least 2 moles of water and at least 2 moles of acid or alkali are employed per mole of starting material.

7. Process according to Claims 1 to 6, characterised in that it is carried out in the presence of an inert organic solvent.

8. Pyrimidinediones and dihydroxypyrimidines of the formulae

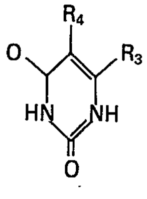 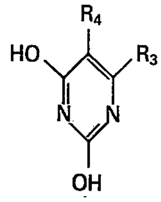 and 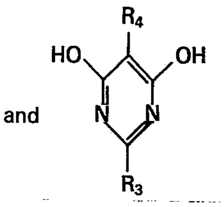

in which

one of the radicals $R_3$ and $R_4$ represents fluorine, chlorine, $CF_3$, $CF_2Cl$, $CCl_2F$, $CHCl_2$, $CHF_2$ or $CHClF$, and the other of the radicals $R_3$ and $R_4$ represents fluorine or chlorine, with the exception of coumpounds in which $R_3$ represents $CF_3$ and $R_4$ represents fluorine.

**Revendications**

1. Procédé de production de pyrimidinediones et de dihydroxypyrimidines de formules

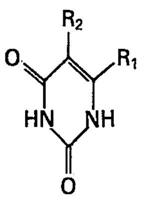 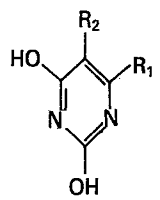 et 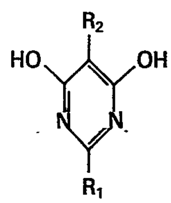

dans lesquelles

$R_1$ représente le fluor, le chlore ou un groupe alkyle en $C_1$ à $C_4$, les atomes d'hydrogène du groupe alkyle étant totalement ou partiellement substitués par du fluor et/ou du chlore et $R_2$ représente l'hydrogène, le fluor ou le chlore, et au cas où $R_1$ est le fluor ou le chlore, $R_2$ représente également un groupe alkyle en $C_1$ à $C_4$, les atomes d'hydrogène du groupe alkyle pouvant être substitués, le cas échéant, entièrement ou partiellement par du fluor et/ou du chlore, caractérisé en ce qu'on hydrolyse avec des acides ou des alcalis aqueux des pyrimidines contenant du fluor et/ou du chlore, de formules

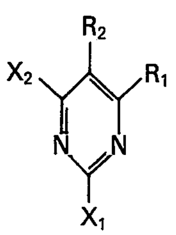 ou 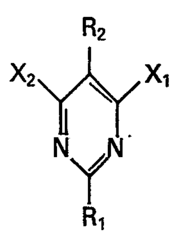

dans lesquelles

$R_1$ et $R_2$ ont la définition indiquée ci-dessus et $X_1$ et $X_2$ représentent, indépendamment l'un de l'autre, du fluor ou du chlore.

2. Procédé suivant la revendication 1, caractérisé en ce qu'on utilise des pyrimidines des formules indiquées dans lesquelles $R_1$ représente du fluor, du chlore, un groupe $CF_3$, $CF_2Cl$, $CCl_2F$, $CCl_3$, $CHCl_2$, $CHF_2$, $CHClF$, $CH_2F$ ou $CH_2Cl$ et $R_2$ représente l'hydrogène, le fluor ou le chlore et, au cas où $R_1$ représente le fluor ou le chlore, $R_2$ représente également le groupe $CF_3$, $CF_2Cl$, $CCl_2$-F, $CCl_3$, $CH_3$, $CHCl_2$, $CHF_2$, $CHClF$, $CH_2F$ ou $CH_2Cl$ et $X_1$ et $X_2$ représentent, indépendamment l'un de l'autre, du chlore.

3. Procédé suivant les revendications 1 et 2, caractérisé en ce qu'on utilise des pyrimidines des formules indiquées dans lesquelles $R_1$ représente le fluor, le chlore ou le groupe $CF_3$ et $R_2$ représente l'hydrogène, le fluor ou le chlore et, au cas où $R_1$ est le fluor ou le chlore, $R_2$ représente également le groupe $CF_3$ et $X_1$ et $X_2$ représentent, indépendamment l'un de l'autre, du fluor ou du chlore.

4. Procédé suivant les revendications 1 à 3, caractérisé en ce qu'il est mis en œuvre à des températures de 0 à 160°C.

5. Procédé suivant les revendications 1 à 4, caractérisé en ce qu'il est mis en œuvre avec addition d'acide sulfurique, d'acide chlorhydrique, d'acide acétique, d'acide nitrique, d'acide phosphorique, de lessive de soude ou de lessive de potasse.

6. Procédé suivant les revendications 1 à 5, caractérisé en ce qu'on utilise au moins 2 moles d'eau et au moins 2 moles d'acide ou d'alcali par mole de matière de départ.

7. Procédé suivant les revendications 1 à 6, caractérisé en ce qu'il est mis en œuvre en présence d'un solvant organique inerte.

8. Pyrimidine-diones et dihydroxypyrimidines de formules

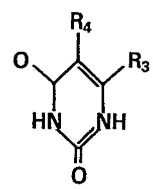 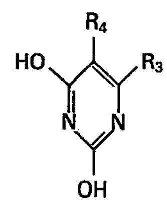 et 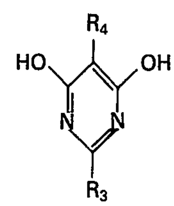

dans lesquelles

l'un des restes $R_3$ et $R_4$ représente le fluor, le chlore, un chlore, un groupe $CF_3$, $CF_2Cl$, $CCl_2F$, $CHCl_2$, $CHF_2$ ou $CHClF$ et l'autre des restes $R_3$ et $R_4$ représente le fluor ou le chlore, à l'exception des composés dans lesquels $R_3$ représente $CF_3$ et $R_4$ représente le fluor.